(19)

Europäisches
Patentamt
European
Patent Office
Office européen
des brevets

(11) **EP 3 624 125 A1**

(12) **EUROPEAN PATENT APPLICATION**

(43) Date of publication:
**18.03.2020   Bulletin 2020/12**

(51) Int Cl.:
**G16H 10/20** (2018.01)          **G16H 40/63** (2018.01)
**G16H 50/30** (2018.01)          **G16H 20/10** (2018.01)

(21) Application number: **18194246.7**

(22) Date of filing: **13.09.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
 • **GAN, Siqi**
  **5656 AE Eindhoven (NL)**

 • **ZHU, Qin**
  **5656 AE Eindhoven (NL)**
 • **JIANG, Jie**
  **5656 AE Eindhoven (NL)**
 • **YIN, Bin**
  **5656 AE Eindhoven (NL)**
 • **HUO, Yong**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54)   **DETERMINING ADHERENCE TO A MEDICATION DOSAGE SCHEME**

(57)     A method and system of predicting or estimating an adherence score representing a subject's adherence to a medication dosage scheme. A subject-reported adherence score is upwardly revised based on application usage information of an application used by the subject to report their adherence. A prediction model may be used to adjust the subject-reported adherence score based on the application usage information.

FIG. 1

EP 3 624 125 A1

Printed by Jouve, 75001 PARIS (FR)

**Description**

FIELD OF THE INVENTION

The present invention relates to the field of determining adherence to a medication dosage scheme, and in particular to determining adherence using subject-reported information.

BACKGROUND OF THE INVENTION

Adherence to a medication dosage scheme is important for post-hospital care or chronic disease management, since poor adherence to long-term therapies leads to poor health outcomes and increased health care cost. There is therefore an increasing interest in accurately assessing adherence to a medication dosage scheme. Adherence assessments provide the foundation for effective treatment planning, medication monitoring, and the need for medical intervention.

However, current methods of ensuring accurate assessment of a subject's adherence to a medication dosage scheme, e.g. regular measurements of drug/metabolite concentration in body fluids or using a medication events monitoring system, are expensive and impractical for routine practice.

Other methods of assessing adherence to a medication dosage scheme, such as using patient self-reported information, are cheaper, more flexible and simpler to implement. However, these methods are less reliable and typically less accurate, as they are reliant on a patient accurately reporting their adherence and can therefore be subjective.

There is therefore a desire to provide an accurate method of assessing adherence to a medication scheme, which is both cheap and relatively simple to implement.

SUMMARY OF THE INVENTION

The invention is defined by the claims.

According to examples in accordance with an aspect of the invention, there is provided a method of calculating an adherence score representative of a predicted adherence of a subject to a medication dosage scheme. The method comprises: obtaining a subject-reported adherence score from an application hosted by a user interface device, wherein the application is accessed by the subject to record their adherence to the medication dosage scheme; obtaining application usage information, the application usage information being indicative of access activity of the application by the subject; and increasing the subject-reported adherence score based on the application usage information to thereby calculate the adherence score representative of the predicted adherence of the subject to the medication dosage scheme.

The present invention relies on an underlying understanding that a subject is liable to underreport their adherence to a medication dosage scheme, for example, due to forgetfulness or misunderstanding when using an application for reporting their adherence. Thus, information about a usage or access activity of an application, used by the subject to log or record their adherence to the medication dosage scheme, can be used to increase an adherence score to account for this underreporting.

This improves an accuracy of the adherence score, so that it more closely aligns with a real or actual adherence of the subject to the medication dosage scheme. Thus, a calculated adherence score is less reliant on the subject accurately reporting their adherence to the medication dosage scheme.

This provides a highly accurate method of assessing an adherence of a subject to a medication dosage scheme, whilst being relatively cheap and simple to implement. As the adherence score is highly accurate, it may therefore be considered to represent the actual adherence of the subject to the medication dosage scheme.

An adherence score, obtained from an application used by a subject to report adherence to the medication dosage scheme, is therefore upwardly corrected using access information of that application. This ensures that failure or forgetfulness of the subject to report their adherence to the medication dosage scheme does not affect the calculated adherence score. Thus, the method accounts for a difference between the subject-reported adherence score and the 'actual' adherence score, by taking into account times when the subject has forgotten or failed to report their taking of the medication.

The access activity may be indicative of, for example, a time since last access of the application, a number of accesses of the application by the subject, a frequency of accesses of the application by the subject, a number of access of the application by the subject within a predetermined time period and so on.

Thus, the access activity may indicate whether a subject has accessed the application in accordance with the medication dosage scheme. For example, if a number of accesses of the application (for self-reporting) within a predetermined period is less than a number of recommended medication dosages (according to the medication dosage scheme) within that predetermined period, then it may be determined that the subject has failed or forgotten to report adherence to the medication dosage scheme - even though the subject may have nonetheless complied with the medication dosage scheme.

In at least one embodiment, the step of increasing the subject-reported adherence score comprises: obtaining a prediction model for predicting, using the subject-reported adherence score and the application usage information, an adherence score representative of the predicted adherence of the subject to the medication dosage scheme; and predicting the adherence score, using the prediction model, based on the subject-reported adherence score and the application usage information.

A prediction model is a method, algorithm or function that receives as input the subject-reported adherence score and application usage information, and outputs a modified/increased adherence score, that more closely represents the actual adherence of the subject to the medication dosage scheme.

Using a prediction model increases an accuracy of the calculated adherence score. In particular the prediction model maybe able to accurately calculate an 'actual' adherence score of the patient based on the subject-reported adherence score and the access activity of the application used by the subject to report their adherence.

The step of obtaining a prediction model may comprise selecting one of a plurality of potential prediction models based on one or more characteristics of the subject and the medication dosage scheme.

By obtaining a prediction model based on one or more characteristics of the patient or the medication dosage scheme, an accuracy of the predicted adherence score may be increased. It has been recognized that patient or medication dosage scheme characteristics may significantly influence a likelihood that a patient will report taking of a medication at any given time, and therefore affect the difference between the subject-reported adherence score and the actual adherence score.

By way of example, if a patient is required to take many medication dosages in a day (due to a diagnosis or medication dosage scheme), they may be more likely to forget or fail to report taking of a medication, e.g. due to fatigue or boredom. By taking characteristics of the patient or medication dosage scheme into account when selecting a prediction model, then these scenarios can be accounted for.

Characteristics of the subject may include, for example, demographic information (e.g. age or gender), diagnosis information or code, treatment information, medical history (e.g. history of cardiovascular problems) or sign/symptom information. Characteristics of the medication dosage scheme may include: frequency of recommended doses, magnitude of recommended doses, number of recommended doses and so on.

Preferably, the step of predicting the adherence score, using the prediction model comprises: determining, based on the application usage information, whether to increase the subject-reported adherence score, and increasing the subject-reported adherence score only when it is determined to increase the subject-reported adherence score.

Thus, the prediction model may comprise determining whether to increase the subject-reported adherence score based on the application usage information. In other words, the prediction model may define whether it is necessary to increase the adherence score in view of the application usage information.

This avoids unnecessary increases to the subject-reported adherence score, e.g. where it considered that the subject has adequately reported their adherence to the medication dosage scheme, to thereby improve an accuracy of the calculated adherence score.

The application usage information may comprises a usage score representative of the access activity of the subject to the application. In such embodiments, the step of determining whether to increase the subject-reported adherence score may comprise determining whether the usage score is above a predetermined score, wherein the predetermined score is defined by the prediction model.

Thus, the prediction model may define whether the subject has adequately reported their adherence to the medication dosage scheme, by setting a minimum access limit to the application. This improves an accuracy of the calculating the modified adherence score.

Optionally, the method further comprises obtaining an actual adherence score representative of an actual adherence of the subject to the medication dosage scheme; and updating the prediction model based on the actual adherence score.

In this way, the prediction model may be adapted or personalized to the subject, rather than being a generic model. This increases the accuracy of the prediction model and thereby of the calculated adherence score.

When the step of determining whether to increase the subject-reported adherence score comprises determining whether a usage score is above a predetermined score, wherein the predetermined score is defined by the prediction model, the step of updating the prediction model may comprise modifying the predetermined score.

The usage score may represent a length of time, e.g. number of days that the subject has failed to access the application to record their adherence to the medication dosage scheme.

In one embodiment, the step of obtaining an actual adherence score comprises obtaining the actual adherence score from an analysis of drug or metabolite concentration of body fluids from the subject.

Such analysis techniques have been identified as providing accurate measurements of a subject's adherence to a medication dosage scheme.

The step of obtaining the subject-reported adherence score may comprise obtaining a count of how many times the subject has indicated, using the application, their compliance with the medication dosage scheme.

The step of obtaining a prediction model may comprise generating a prediction model. A method of generating a prediction model comprises: obtaining a dataset of data entries, each data entry associated with a subject and a medication dosage scheme, and each data entry comprising a historic self-reported adherence score, historic application usage information and a historic actual adherence score; for each data entry, predicting, using a generic prediction model, a historic adherence score representative of a historic predicted adherence of the subject associated with the data entry to a medication dosage scheme, using the historic self-reported adherence information and the historic application usage information; and modifying the generic prediction model using the historic adherence score and the historic actual adherence score of each data entry to thereby obtain the prediction model.

The step of modifying the generic prediction model preferably comprises iterative steps of: for each data entry, calculating a difference between the historic adherence score and the historic actual adherence score; summing the calculated differences of each data entry, to generate a summed total; and modifying the generic prediction model to reduce the summed total in a subsequent iteration.

In embodiments, the historic application usage information comprises a historic usage score representative of the access activity to an application accessed by the subject, associated with the data entry, to record their adherence to the medication dosage scheme. In further examples, the step of predicting, using the prediction model, comprises: determining whether the historic usage score is greater than a predetermined value, the predetermined value being set by the generic prediction model; and increasing the historic self-reported adherence score only if the usage score is greater than a predetermined value to thereby generate the historic adherence score; and the step of modifying the generic prediction model comprises modifying the predetermined value.

The step of obtaining a dataset of data entries may comprise obtaining the dataset based on one or more characteristics of the subject and the medication dosage scheme.

Preferably, the step of obtaining the subject-reported adherence score comprises obtaining a count of how many times the subject has indicated, using the application, their compliance with the medication dosage scheme.

The subject-reported adherence score may therefore be indicative of a number of time the subject has reported their adherence with the medication dosage scheme. This provides a simple but effective method of indicating the subject's compliance (e.g. as the greater the number, the more closely the subject is likely to have complied with the medication dosage scheme).

The step of obtaining a subject-reported adherence score may further comprise dividing the count by the number of dosages recommended by the medication dosage scheme.

Thus, the subject-reported adherence score may represent a proportion of the recommended dosages of the medication dosage scheme to which the subject has indicated their adherence. This increases a conceptual understanding of the relative adherence of the subject to the medication dosage scheme.

The method may further comprise generating an alert when the calculated adherence score is below a predetermined value.

Thus, an alert may be generated in response to a determination that the subject has not adequately adhered to the medication dosage scheme. This may allow, for example, for medical intervention to avoid medical condition deterioration.

According to examples in accordance with another aspect of the invention, there is provided a computer program comprising code means for implementing any previously described method when said program is run on a computer.

According to examples in accordance with yet another aspect of the invention, there is provided a system for calculating an adherence score representative of a predicted adherence of a subject to a medication dosage scheme. The system comprises an obtaining unit adapted to obtain: a subject-reported adherence score from an application for a user interface, wherein the application is accessed by the subject to record their adherence to the medication dosage scheme; and application usage information indicative of access activity of the application by the subject. The system also comprises a calculating unit adapted to increase the subject-reported adherence score based on the application usage information to thereby generate an adherence score representative of the predicted adherence of the subject to the medication dosage scheme.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a flow-chart illustrating a method according to an embodiment;
Figure 2 illustrates a user interface for hosting an application to be used by a subject to report adherence information;

Figure 3 illustrates steps for obtaining a prediction model according to an embodiment;
Figure 4 illustrates a process of generating a prediction model according to an embodiment;
Figure 5 illustrates steps for modifying a prediction model according to an embodiment; and
Figure 6 illustrates a system according to an embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a method and system of predicting or estimating an adherence score representing a subject's adherence to a medication dosage scheme. A subject-reported adherence score is upwardly revised based on application usage information of an application used by the subject to report their adherence. A prediction model may be used to adjust the subject-reported adherence score based on the application usage information.

Embodiments are at least partly based on the realization that subjects under-report their adherence to a medication dosage scheme, due to forgetfulness or misunderstanding. By using access activity information of an application used by the subject to report their adherence, a subject-reported adherence score can be upwardly corrected to account for a subject's failure to report adherence to a medication dosage scheme.

Illustrative embodiments may, for example, be employed in chronic disease management solutions, where subjects are required to take medication for an extended period of time, or in other long-term medication dosage systems. In this way, adherence to a chronic disease medication scheme can be accurately assessed, and failure by a subject to adhere to the scheme accurately identified.

A "medication dosage scheme" identifies how many times (e.g. over a set period of time) a subject should have taken a particular medication.

An "adherence score" is a measure of whether the subject has adhered to the medication dosage scheme, i.e. whether they have taken the correct number of doses of medication. In particular, the adherence score may represent a measure of how closely the subject has adhered to the medication dosage scheme over a predetermined period of time, e.g. a day or a week.

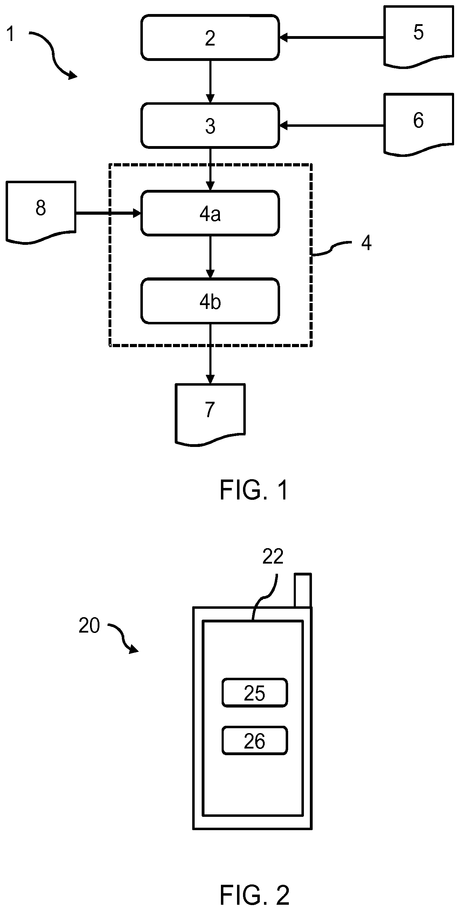

Figure 1 illustrates a method 1 of calculating an adherence score representative of a predicted adherence of a subject to a medication dosage scheme.

The method 1 comprises a step 2 of obtaining a subject-reported adherence score 5.

The subject-reported adherence score 5 is provided by an application hosted by a user interface device, such as a mobile phone or tablet. As will be later exemplified, the user interface provides an application or program through which a subject is able to record their adherence to a medication dosage scheme.

The method then moves to a step 3 of obtaining application usage information 6. The application usage information indicates an access activity of the application/program by the subject/user.

Subsequently, the method moves to a step 4 of increasing the subject-reported adherence score 5 based on the application usage information 6. By increasing the subject-reported adherence score in step 4, an (predicted) adherence score 7 representative of the predicted adherence of the subject to the medication dosage scheme can be obtained.

The adherence score 7 generated in step 3 is therefore a predicted or estimated adherence score, and maybe alternatively labelled an "adjusted adherence score". The adherence score 7 can be used in place of the "actual adherence score" (which represents an extremely accurate assessment of the subject's adherence), e.g. for the triggering of alerts or informing a clinician of the subject's adherence.

The present invention relies on the understanding that a subject is likely to under-report their adherence to a medication scheme, e.g. due to forgetfulness.

By way of example, if the application usage information 6 indicates that a subject has not accessed the application (for reporting adherence) for a predetermined length of time, the subject-reported adherence score 5 may be increased by a predetermined value or percentage to account for missing reports by the subject.

In a preferred embodiment, and as illustrated, the step 4 of increasing the subject-reported adherence score may comprise a number of sub-steps. In an embodiment, step 4 comprises a first sub-step 4a of obtaining a prediction model 8 for predicting, based on the subject-reported adherence score and the application usage information, an adherence score 7. The prediction model 8 is used in sub-step 4b to predict the adherence score.

Various prediction models are envisaged by the present invention, of which a first example is hereafter described.

In this first example, a subject-reported adherence score is obtained using a self-reported electronic diary or log approach.

Figure 2 illustrates a user interface 20, such a mobile phone, which can act as an electronic diary for the subject to record their adherence to a medication dosage scheme. The user interface comprises a touch-sensitive screen 22 that displays an application. The application provides two buttons, a first button 25 and a second button 26.

To record adherence, the application allows a subject to indicate, via the first button 25, whether they have taken ("Yes") a medication or whether, via the second button, they have not taken ("No") the medication. Thus, adherence or non-adherence to a medication dosage scheme can be logged by a subject clicking a "Yes" button 25 or a "No" button 26 provided by the user interface 20. In this way, the subject reports whether they have adhered to a medication dosage scheme.

A subject-reported adherence score $S_{sub}$ can be calculated using the follow equation:

$$S_{sub} = \frac{N_{yes}}{N_{dosage}} \qquad (1)$$

where $N_{yes}$ represents a number of times a subject has indicated they have taken a medication and $N_{dosage}$ represents a number of times the subject ought to have taken the medication according to a medication dosage scheme. $N_{no}$ (used later) represents a number of times a subject has indicated they have not taken a medication.

Thus, obtaining the subject-reported adherence score $S_{sub}$ comprise obtaining a count $N_{yes}$ of how many times the subject has indicated, using the application, their compliance with the medication dosage scheme.

In other words, referring back to Figure 1, the step 2 of obtaining the subject-reported adherence score 5 may comprise obtaining at least a count $N_{yes}$ of how many times the subject has logged, using an application, their compliance with the medication dosage scheme. The step 2 may further comprise determining what proportion of the recommended number of dosages that the subject has indicated their compliance with the medication dosage scheme.

In step 4, subject-reported adherence score is adjusted based on application usage information to obtain an (estimated) adherence score $S_{ad}$. In particular, the prediction model 8 increases the subject-reported adherence score $S_{sub}$ using a predetermined value Nu, where the predetermined value Nu is determined by the application usage information 6.

In the first example, the application usage information 6 comprises a usage score. The usage score is representative of the access activity of the subject to the application. A usage score may, for example, indicate a length of time, such as a number of days $N_D$, since the application was last accessed by the subject.

In response to the length of time $N_t$ being less than a predetermined score p, the predetermined value $N_U$ may be set to 0. In response to the length of time Nt being greater than or equal to the predetermined score p, the predetermined value $N_U$ may be set to a second predetermined value $n_u$. Thus:

$$N_u \begin{cases} 0, & if\ N_t < p \\ n_u, & if\ N_t \geq p \end{cases} \qquad (2)$$

In this way, the application usage information defines whether or not to increase the subject-reported adherence score. The prediction model can define the value of the predetermined score p.

The second predetermined value $n_u$ may be equal to the number of times that it is unknown whether the subject has taken a medication. Thus, the second predetermined value $n_u$ may represent the magnitude of a difference between the number of times $N_{dosage}$ a subject should have taken the medication according to the medication dosage scheme and the sum of the number of times $N_{yes}$ the subject has indicated they have taken a medication and the number of time $N_{no}$ the subject has indicated they have not taken a medication. Thus, the second predetermined value $n_u$ can be calculated as follows:

$$n_u = N_{dosage} - N_{yes} - N_{no} \qquad (3)$$

However, the second predetermined value $n_u$ may be replaced by any suitable predetermined value, or may be otherwise defined by the prediction model.

The (estimated) adherence score $s_{ad}$ can then be calculated using the following equation:

$$S_{ad} = S_{sub} + \frac{N_U}{N_{dosage}} = \frac{N_{yes}}{N_{dosage}} + \frac{N_U}{N_{dosage}} = \frac{N_S + N_U}{N_{dosage}} \qquad (4)$$

In this way, the estimated adherence score $S_{ad}$ depends upon the subject-reported adherence score $S_{sub}$ and the application usage information, that defines the predetermined value $N_U$.

As the predetermined value Nu may be either 0 or the second predetermined value $n_u$, the proposed method may increase the subject-reported adherence score (by the second predetermined value $n_U$) only when it is determined to increase the subject-reported adherence score.

In preferable examples, different prediction models define different values for the predetermined score p.

Thus, using a prediction model in sub-step 4b may comprise determining whether to increase the subject-reported adherence score (i.e. equation 2), and increasing the subject-reported adherence score (i.e. equation 4) only when it is determined to increase the subject-reported adherence score.

The prediction model used in step 4 may define the equations 2-4. In other words, the prediction model determines how the subject-reported adherence score $S_{sub}$ is adjusted based on the application usage information.

In particular, when the application usage information comprises a usage score representative of the access activity of the subject to the application, the step of determining whether to increase the subject-reported adherence score may comprise determining whether the usage score is above a predetermined score, wherein the predetermined score is defined by the prediction model.

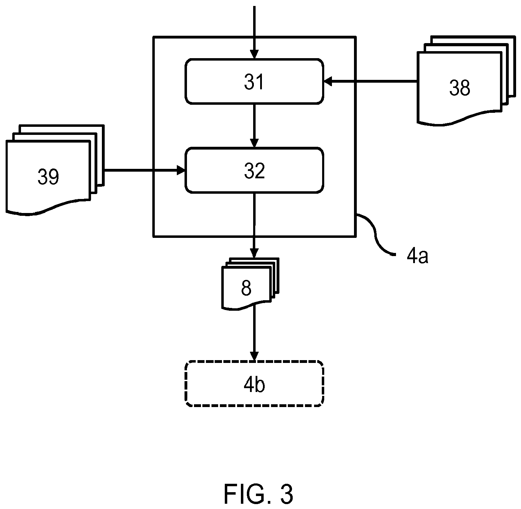

Figure 3 illustrates an embodiment of the sub-step 4a of obtaining a prediction model according to an embodiment of the invention.

The sub-step 4a comprises a sub-step 31 of obtaining one or more characteristics 38 of the subject and/or the medication dosage scheme (associated with the subject).

The sub-step 4a further comprises a sub-step 32 of selecting one of a plurality of prediction models 39 based on the one or more characteristics of the subject and the medication dosage scheme.

Thus, sub-step 4a comprises selecting a prediction model, from a number of potential prediction models, based on the characteristic(s) of a subject and/or the associated medication dosage scheme.

In other words, there may be a plurality of prediction models, each associated with a different cohort of subjects and/or medication dosage schemes. Step 32 comprises selecting the prediction model associated with a cohort must similar to the subject under investigation. By way of example, sub-step 4b may comprise performing a minimum distance algorithm.

Sub-step 4a then outputs the selected prediction model 8 for use in sub-step 4b of using the prediction model to predict the adherence score.

Characteristics of the subject may include, for example, demographic information (e.g. age or gender), diagnosis information or code, treatment information, medical history (e.g. history of cardiovascular problems) or sign/symptom information. Characteristics of the medication dosage scheme may include: frequency of recommended doses, magnitude of recommended doses, number of recommended doses and so on.

Thus, sub-step 4a may comprise selecting one of a plurality 39 of potential prediction models based on one or more characteristics of the subject and the medication dosage scheme.

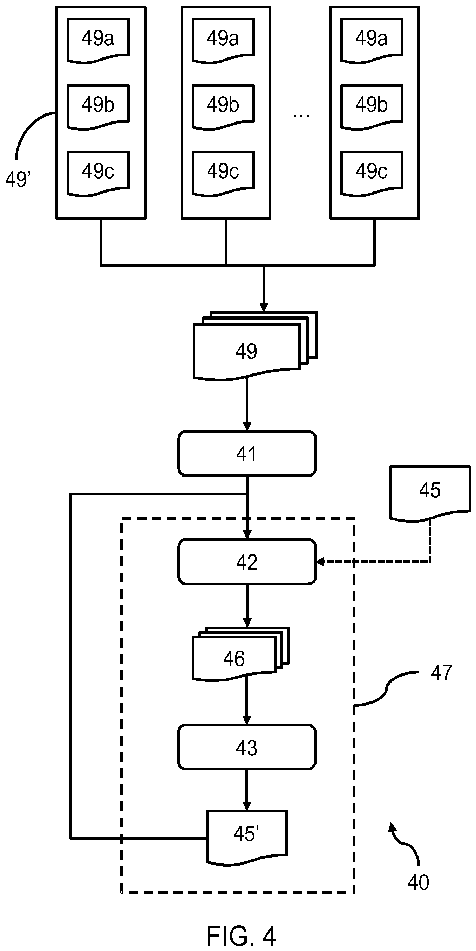

Figure 4 illustrates a method 40 of generating a prediction model 45', for use in step 4 of method 1.

The method 40 comprises a step 41 of obtaining a dataset 49 of data entries 49', each of which is associated with a subject and a medication dosage scheme. A data entry 49' comprises a historic self-reported adherence score 49a, historic application usage information 49b and a historic actual adherence score 49c.

Each historic element of the data entry 49' represents previously reported or known information, and are temporally aligned with one another. Each data entry may be associated, for example, with a different period of time over which adherence is determined and/or a different subject.

By way of example, the dataset may comprise: a first plurality of data entries associated with a first subject, each data entry of the plurality being associated with a different period of time over which adherence is determined; and a second plurality of data entries associated with a second subject, each data entry of the plurality being associated with a different period of time of which adherence is determined. The dataset may comprise any number of such pluralities, each plurality associated with different subjects.

Thus, the historic self-reported adherence score 49a may indicate a historic record of a number of times a subject indicated their adherence to a medication dosage scheme via iteration with an application of a user interface (e.g. using a "Yes" button).

A historic actual adherence score represents an actual adherence of the subject to the medication dosage scheme. Such an actual adherence score may be obtained, for example, by performing a blood or fluid concentration analysis on the subject (to identify whether they have taken the correct number of medication doses).

The method 40 further comprises iteratively performing a step 41 of using a generic prediction model 45 to predict a historic adherence score for a data entry, where the historic adherence score is representative of a predicted adherence of the subject (associated with the data entry) to the medication dosage scheme. To predict the historic adherence score, the generic prediction model processes a historic self-reported adherence score 49a and historic application usage information 49b of a data entry.

Methods of predicting the historic adherence score maybe substantially identical to the steps of predicting an adherence score described with reference to Figures 1 to 2.

The step 42 is performed on each data entry 49' of the dataset 49, so that a respective plurality 46 of historic adherence scores are generated. Each historic adherence score 46 is thereby associated with a respective data entry 49'.

The method 40 further comprises a step 43 of modifying the generic prediction model 45 using the historic adherence score 46 and historic actual adherence score 49c of each data entry 49'.

In particular, step 43 comprises modifying the generic prediction model to try and minimize the difference between the historic (predicted) adherence score 46 and the historic actual adherence score 49c of each data entry 49'.

Steps 42 and 43 may be iteratively repeated, using the modified prediction model 45' each time, to determine a result of the modification and determine how or whether to further modify the generic prediction model. Thus, steps 42 and 43 acts as a modification process 47 to modify the generic prediction model with an aim to minimize a difference between historic adherence scores and historic actual adherences scores of the dataset 49.

After a predetermined number of iterations of the modification process 47, or when the difference between historic adherence scores and historic actual adherence scores is below a predetermined value, the iterative repetition of the modification process 47 may stop.

In a first example, the prediction model generated using the modification process 47 is structured similarly to the prediction model described with reference to equation 2-4. That is, the prediction model may define a magnitude of a predetermined score p. In further examples, the prediction model may define a magnitude of the second predetermined value $n_u$

Thus, step 43 may comprise modifying the magnitude of the predetermined score p and/or the second predetermined value $n_u$. The modification score 47 may therefore comprise modifying the predetermined score p defined by the generic prediction model in an attempt to reduce a difference between the historic adherence score 46 and the historic actual adherence score 49c of each data entry.

In particular, the modification process 47 aims to perform the following optimization or minimization process:

$$\min \sum_1^N |S_{had} - S_{hac}| \tag{5}$$

where N is the number of data entries, $S_{had}$ is a historic adherence score and $S_{hac}$ is a historic actual adherence score. $S_{had}$ can be calculated in an identical manner to $S_{ad}$ of equation 4, but using the historic self-reported adherence score and historic application usage information.

Methods of adjusting a prediction model to minimize a difference between a historic adherence score and a historic actual adherence score will be well known to the skilled person. In other words, the method 40 of generating a prediction model may comprise minimizing a difference between historic estimated adherence scores and associated historic actual adherence scores.

The method 40 may be performed on a plurality of different datasets to generate different prediction models 45' for use with the method of calculating an adherence score representative of a predicted adherence of a subject to a medication dosage scheme.

Different datasets may be associated with different cohorts of data entries (and thereby subjects or medication dosage schemes). Thus, data entries in single dataset may be associated with subjects sharing similar characteristics, such as diagnosis, treatment methodology and/or medication dosage schemes.

In this way, a plurality of different prediction models can be generated, each prediction model being associated with a different cohort of subjects and/or medication dosage schemes.

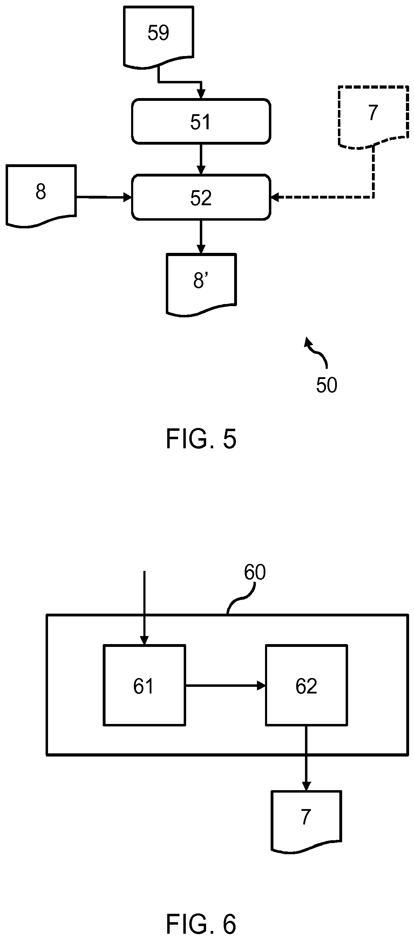

Figure 5 illustrates a further process 50 for improving a method according to an embodiment.

The further process comprises a step 51 of obtaining an actual adherence score 59 representative of an actual adherence of the subject to the medication dosage scheme.

The actual adherence score may, for example, be obtained from an analysis of drug or metabolite concentration of body fluids from the subject. In other examples, the actual adherence score may be obtain from a medication survey (e.g. checking how many medication doses the patient has left). Thus, the actual adherence score accurately reflects

8

the adherence of a subject to a medication dosage scheme.

The further process 50 further comprises a step 52 of updating the prediction model 8 based on the actual adherence score 59. Thus, an updated prediction model 8' is output.

Thus, the further process aim to update or personalize the prediction model based on the actual adherence of the subject to a medication dosage scheme. This results in a more accurate and bespoke prediction model to be used for estimating the adherence of a subject to a medication dosage scheme.

The method of updating the prediction model based on the actual adherence of the subject may be similar to the method of adjusting the prediction model described with reference to Figure 4.

In other words, step 51 may comprise an iterative process of determining a difference between the actual adherence score 59 and the adherence score 7, and altering the prediction model with an aim to reduce the determined difference.

In the described examples, the application usage information indicates a length of time (e.g. number of days) that the application has not been accessed. However, in other examples, the application usage information may indicate an amount of time the subject accesses the application, a frequency of the subject accessing the application, a relationship between accessing of the app and an indication by the subject whether they have adhered to the medication dosage scheme and so on.

Other prediction models will be apparent to the skilled person. For example, a prediction model may define an algorithm to be apply to the self-reported adherence score, where the application usage information defines a variable of the algorithm to be applied, to thereby generate the adherence score.

Figure 6 illustrates a system 60 for calculating an adherence score representative of a predicted adherence of a subject to a medication dosage scheme. The system 60 comprises an obtaining unit 61 and a calculating unit 62.

The obtaining unit 61 is adapted to obtain: a subject-reported adherence score from an application for a user interface, wherein the application is accessed by the subject to record their adherence to the medication dosage scheme; and application usage information indicative of access activity of the application by the subject.

The calculating unit 62 is adapted to increase the subject-reported adherence score based on the application usage information to thereby generate an adherence score representative of the predicted adherence of the subject to the medication dosage scheme.

Embodiments make use of a system. The system can be implemented in numerous ways, with software and/or hardware, to perform the various functions required. A processor is one example of a system which employs one or more microprocessors that may be programmed using software (e.g., microcode) to perform the required functions. A system may however be implemented with or without employing a processor, and also may be implemented as a combination of dedicated hardware to perform some functions and a processor (e.g., one or more programmed microprocessors and associated circuitry) to perform other functions.

Examples of system components that may be employed in various embodiments of the present disclosure include, but are not limited to, conventional microprocessors, application specific integrated circuits (ASICs), and field-programmable gate arrays (FPGAs).

In various implementations, a processor or system may be associated with one or more storage media such as volatile and non-volatile computer memory such as RAM, PROM, EPROM, and EEPROM. The storage media may be encoded with one or more programs that, when executed on one or more processors and/or systems, perform the required functions. Various storage media may be fixed within a processor or system or may be transportable, such that the one or more programs stored thereon can be loaded into a processor or system.

Thus, the present invention may be a system, a method, and/or a computer program product. The computer program product may include a computer readable storage medium (or media) having computer readable program instructions thereon for causing a processor to carry out aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. Each block of the block diagrams and/or flowchart illustration, and combinations of blocks in the block diagrams and/or flowchart illustrations, can be implemented by special purpose hardware-based systems that perform the specified functions or acts or carry out combinations of special purpose hardware and computer instructions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.  A method (1) of calculating an adherence score (7) representative of a predicted adherence of a subject to a medication dosage scheme, the method comprising:

    obtaining (2) a subject-reported adherence score (5) from an application hosted by a user interface device (20), wherein the application is accessed by the subject to record their adherence to the medication dosage scheme;
    obtaining (3) application usage information (6), the application usage information being indicative of access activity of the application by the subject; and
    increasing (4) the subject-reported adherence score based on the application usage information to thereby calculate the adherence score representative of the predicted adherence of the subject to the medication dosage scheme.

2.  The method of claim 1, wherein the step (4) of increasing the subject-reported adherence score comprises:

    obtaining (4a) a prediction model (8) for predicting, using the subject-reported adherence score (5) and the application usage information (6), an adherence score (7) representative of the predicted adherence of the subject to the medication dosage scheme; and
    predicting (4b) the adherence score, using the prediction model, based on the subject-reported adherence score and the application usage information.

3.  The method of claim 2, wherein the step (4a) of obtaining a prediction model comprises selecting (32) one of a plurality of potential prediction models based on one or more characteristics of the subject and the medication dosage scheme.

4.  The method of any of claims 2 or 3, wherein the step (4b) of predicting the adherence score, using the prediction model comprises:

    determining, based on the application usage information, whether to increase the subject-reported adherence score, and
    increasing the subject-reported adherence score only when it is determined to increase the subject-reported adherence score.

5.  The method of claim 4, wherein:

    the application usage information comprises a usage score representative of the access activity of the subject to the application; and
    the step (4b) of determining whether to increase the subject-reported adherence score comprises determining whether the usage score is above a predetermined score, wherein the predetermined score is defined by the prediction model.

6.  The method of any of claims 2 to 5, further comprising:

    obtaining (51) an actual adherence score representative of an actual adherence of the subject to the medication dosage scheme; and
    updating (52) the prediction model based on the actual adherence score.

7.  The method of claim 6, when dependent on claim 5, wherein the step (52) of updating the prediction model comprises modifying the predetermined score.

8.  The method of claim 6 or 7, wherein the step (51) of obtaining an actual adherence score comprises obtaining the actual adherence score from an analysis of drug or metabolite concentration of body fluids from the subject.

9. The method of any of claims 2 to 8, wherein the step (2) of obtaining the subject-reported adherence score comprises obtaining a count of how many times the subject has indicated, using the application, their compliance with the medication dosage scheme.

10. The method of any of claims 2 to 9, wherein the step (4a, 40) of obtaining a prediction model comprises generating a prediction model by:

obtaining (41) a dataset (49) of data entries, each data entry (49') associated with a subject and a medication dosage scheme, and each data entry comprising a historic self-reported adherence score (49a), historic application usage information (49b) and a historic actual adherence score (49c);
for each data entry, predicting (41), using a generic prediction model, a historic adherence score (46) representative of a historic predicted adherence of the subject associated with the data entry to a medication dosage scheme, using the historic self-reported adherence information and the historic application usage information; and
modifying (43) the generic prediction model using the historic adherence score and the historic actual adherence score of each data entry to thereby obtain the prediction model.

11. The method of claim 10, wherein the step of modifying (43) the generic prediction model comprises iterative steps of:

for each data entry, calculating a difference between the historic adherence score and the historic actual adherence score;
summing the calculated differences of each data entry, to generate a summed total; and
modifying the generic prediction model to reduce the summed total in a subsequent iteration.

12. The method of claim 11, wherein:

the historic application usage information (49b) comprises a historic usage score representative of the access activity to an application accessed by the subject, associated with the data entry, to record their adherence to the medication dosage scheme;
the step of predicting, using the prediction model, comprises:

determining whether the historic usage score is greater than a predetermined value, the predetermined value being set by the generic prediction model; and
increasing the historic self-reported adherence score only if the usage score is greater than a predetermined value to thereby generate the historic adherence score; and

the step of modifying the generic prediction model comprises modifying the predetermined value.

13. The method of any of claims 10 to 12, wherein the step (41) of obtaining a dataset (49) of data entries (49') comprises obtaining the dataset based on one or more characteristics of the subject and the medication dosage scheme.

14. A computer program comprising code means for implementing the method of any one of claims 1 to 13 when said program is run on a computer.

15. A system (60) for calculating an adherence score (7) representative of a predicted adherence of a subject to a medication dosage scheme, the system comprising:

an obtaining unit (61) adapted to obtain:

a subject-reported adherence score from an application for a user interface, wherein the application is accessed by the subject to record their adherence to the medication dosage scheme; and
application usage information indicative of access activity of the application by the subject, and

a calculating unit (62) adapted to increase the subject-reported adherence score based on the application usage information to thereby generate an adherence score representative of the predicted adherence of the subject to the medication dosage scheme.

FIG. 1

FIG. 2

FIG. 3

FIG. 4

FIG. 5

FIG. 6

Europäisches
Patentamt

European
Patent Office

Office européen
des brevets

## EUROPEAN SEARCH REPORT

Application Number

EP 18 19 4246

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/052465 A1 (MADAN ANMOL [US] ET AL) 20 February 2014 (2014-02-20) | 1-7,9-15 | INV.<br>G16H10/20 |
| Y | * paragraphs [0043], [0057], [0077]; figure 1a * | 8 | G16H40/63<br>G16H50/30<br>G16H20/10 |
| | ----- | | |
| Y | WO 2018/001856 A1 (NOVO NORDISK AS [DK]) 4 January 2018 (2018-01-04) * page 19, line 17 - line 33; figures 1,5 * | 8 | |
| | ----- | | |

TECHNICAL FIELDS
SEARCHED (IPC)

G16H

The present search report has been drawn up for all claims

| Place of search | Date of completion of the search | Examiner |
|---|---|---|
| Munich | 22 February 2019 | Samulowitz, Michael |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 18 19 4246

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2019

| Patent document cited in search report | Publication date | Patent family member(s) | Publication date |
|---|---|---|---|
| US 2014052465 A1 | 20-02-2014 | CN 104584017 A | 29-04-2015 |
| | | EP 2884888 A2 | 24-06-2015 |
| | | JP 6185066 B2 | 23-08-2017 |
| | | JP 2015529359 A | 05-10-2015 |
| | | US 2014052465 A1 | 20-02-2014 |
| | | US 2014052474 A1 | 20-02-2014 |
| | | US 2014052475 A1 | 20-02-2014 |
| | | US 2018052974 A1 | 22-02-2018 |
| | | US 2018068085 A1 | 08-03-2018 |
| | | US 2018342327 A1 | 29-11-2018 |
| | | WO 2014028888 A2 | 20-02-2014 |
| WO 2018001856 A1 | 04-01-2018 | NONE | |

EPO FORM P0459

For more details about this annex : see Official Journal of the European Patent Office, No. 12/82